# EUROPÄISCHE PATENTANMELDUNG

(11) **EP 1 845 166 A1**
(43) Veröffentlichungstag der Anmeldung: **17.10.2007**
(21) Anmeldenummer: 06007829.2
(22) Anmeldetag: 13.04.2006
(51) Int. Cl.: C12Q 1/68

(54) **Neue Nukleinsäuresonden mit kovalent gebundenen, interkalierenden Fluoreszenzfarbstoffen zur Hybridisierung an Nukleinsäureproben**

(71) Anmelder: BioSpring GmbH, 60386 Frankfurt (DE)
(72) Erfinder: Aygün, Hüseyin, Dr., 60322 Frankfurt am Main (DE)
(74) Vertreter: Keller, Günter

(57) **Zusammenfassung**

Die vorliegende Erfindung betrifft Nukleinsäuresonden aus DNA, RNA, LNA, CNA, HNA oder ANA oder FANA, oder Chimären aus den aufgeführten Typen, wobei die Nukleinsäuresonden weitere Modifikationen am Zucker, an der Base oder am Phosphatrückgrat aufweisen können, und bei denen eine oder mehrere Basenpositionen durch eine homologe Base ersetzt wurden, an die ein doppelstrang-bindender Fluoreszenzfarbstoff kovalent über eine Linkereinheit gebunden ist.

Des Weiteren betrifft die vorliegende Erfindung ein Verfahren zur Hybridisierung von Nukleinsäureproben. In dem Verfahren zur Hybridisierung kann optional ein Amplifizierungsschritt der Nukleinsäureprobe erfolgen. Die vorliegende Erfindung stellt aber auch ein Verfahren zur direkten Detektion von Nukleinsäuren bzw. deren Derivaten zur Verfügung, ohne dass ein Amplifizierungsschritt notwendig ist.

SEQUENCE LISTING

<110>  BioSpring GmbH

<120>  Neue Nukleinsäuresonden mit kovalent gebundenen, interkalierenden
        Fluoreszenzfarbstoffen zur Hybridisierung an Nukleinsäureproben

<130>  EP 06007829.2

<160>  8

<170>  PatentIn version 3.3

<210>  1
<211>  24
<212>  DNA
<213>  Artificial

<220>
<223>  Primer

<220>
<221>  misc_feature
<222>  (7)..(7)
<223>  Farbstoffmarkierung mit SYBR

<400>  1
agcggataac aatttcacac agga                                              24

<210>  2
<211>  24
<212>  DNA
<213>  Artificial

<220>
<223>  Gegenstrang mit 0 Fehler

<400>  2
tcctgtgtga aattgttatc cgct                                              24

<210>  3
<211>  24
<212>  DNA
<213>  Artificial

<220>
<223>  Gegenstrang mit 1 Fehler

<400>  3
tcctgtgtga atttgttatc cgct                                              24

<210>  4
<211>  24
<212>  DNA
<213>  Artificial

<220>
<223>  Gegenstrang mit 2 Fehler

<400> 4
tcctgtctga aattgatatc cgct 24

<210> 5
<211> 24
<212> DNA
<213> Artificial

<220>
<223> Gegenstrang mit 3 Fehler

<400> 5
tcgtgtgtca aattgttttc cgct 24

<210> 6
<211> 24
<212> DNA
<213> Artificial

<220>
<223> Gegenstrang mit 4 Fehler

<400> 6
tcccgtgtgc aaatgttagc cgct 24

<210> 7
<211> 24
<212> DNA
<213> Artificial

<220>
<223> M13fwd

<400> 7
cgccagggtt ttcccagtca cgac 24

<210> 8
<211> 24
<212> DNA
<213> Artificial

<220>
<223> M13rev

<400> 8
agcggataac aatttcacac agga 24

## Beschreibung

Die vorliegende Erfindung betrifft Nukleinsäuresonden aus DNA, RNA, LNA (Locked Nukleinsäuren), CNA (Cyclohexanyl Nukleinsäure), HNA (Hexitol Nukleinsäuren), ANA (Arabino-Nukleinsäuren) oder FANA (2' Desoxy- 2' fluoro-Arabinonukleinsäuren), oder Chimären aus den aufgeführten Typen, wobei die Nukleinsäuresonden weitere Modifikationen am Zucker, an der Base oder am Phosphatrückgrat aufweisen können, und bei denen eine oder mehrere Basenpositionen durch eine homologe Base ersetzt wurden, an die ein doppelstrang-bindender (interkalierender) Fluoreszenzfarbstoff kovalent über eine Linkereinheit gebunden ist.

Des Weiteren betrifft die vorliegende Erfindung ein Verfahren zur Hybridisierung einer Nukleinsäuresonde mit einer Nukleinsäureprobe. In dem Verfahren zur Hybridisierung kann ein Amplifizierungsschritt der Nukleinsäureprobe erfolgen, oder die Nukleinsäureprobe wird direkt detektiert, ohne dass ein Amplifizierungsschritt durchgeführt wird.

Grundlage für die vorliegende Erfindung ist eine neuartige Markierungsmöglichkeit von Nukleinsäuren mittels spezieller Farbstoffe, so genannter Fluoreszenzinterkalatoren. Die Ausbildung eines Doppelstranges führt zum Interkalieren/Binden eines mit einem der Nukleinsäurestränge kovalent verbundenen Interkalators, der daraufhin seine Fluoreszenzeigenschaften drastisch ändert und dadurch eine spezifische Detektion ermöglicht.

Das PCR Verfahren ist eine weit verbreitete Methode zum Nachweis von spezifischen DNA-Abschnitten. In Kombination mit real-time PCR-Geräten werden dabei Fluoreszenzmarkierte Sonden eingesetzt, um eine rasche Detektion, ohne gelelektrophoretische Auftrennung zu ermöglichen. Es wird in Abhängigkeit des eingesetzten Sondentyps eine Zu- bzw. Abnahme von Fluoreszenz oder Fluoreszenzpolarisation (Wong et al, BioTechniques 2005, 39, 75 - 85 und EP0678581) gemessen.

Bei den Fluoreszenzmessungen kommen häufig aufwendige Sondentypen mit zwei Farbstoffmarkierungen zum Einsatz, die aus einem Farbstoffmolekül und einem sogenannten Quencher bestehen, der das emmitierte Licht des Farbstoffmoleküles absorbieren kann (TaqMan, molecular beacon, ARMS, Scorpions etc.). Je nach möglicher Konformation oder Integrität (Nuklease-abhängig) des Primers wird die Fluoreszenz des Farbstoffmoleküles durch den Quencherfarbstoff absorbiert, oder wenn Farbstoff und Quencher räumlich weit genug voneinander getrennt vorliegen, durch entsprechende Detektoren aufgezeichnet (FRET-Systeme, z.B. DE19755642 und Tyagi et al., Nat. Biotechnol. 2000, 18, 1191 - 1196).

Aus der WO9729210 ist bekannt, dass für diese Art von Sonden, jeweils eine Kombination von 2 Farbstoffen benötigt wird, die auch auf zwei getrennten, komplementären Primern vorliegen können. Eine weitere Möglichkeit in diesen Systemen ist der partielle Abbau des farbstoffmarkierten Primers durch die Exonukleaseaktivität der Polymerase im Laufe der PCR-Reaktion, sodass der freigesetzte Farbstoff, der hierdurch seine Emissionseigenschaften ändert, quantifiziert werden kann.

Ein weiteres Prinzip, das mit nur einer Farbstoffmarkierung auskommt, geht von einer veränderten Umgebung des Farbstoffes nach dem Einbau des Primers in einen Duplexstrang aus (LUX^{®} etc., z.B. Chen, et al., J Virol Methods, 2004, 122, 57 - 61; Nazarenko et al., Nucl. Acid Res. 2000, 30, e37 und Nazarenko et al., Nucl. Acid Res. 2000, 9, 2089-2195). Durch die Veränderung der Nachbarschaft kommt es dann zu einer Veränderung der Fluoreszenz, die wieder ermittelt werden kann. Dabei wird die fluoreszenz-quenchende Eigenschaft der Nukleobase G ausgenutzt, oder es werden in unmittelbarer Nachbarschaft zum Fluoreszenzfarbstoff spezielle Basenmodifikationen in die Primersequenz mit eingebracht, die die Fluoreszenzeigenschaften beeinflussen (z.B. US 6,635,427 "**Wittwer**, C. et al. (2002); Single-labeled oligonucleotide probes WO 2002/014555"; Vaughn et al., American J. Pathology (AJP) 2003, 163, 29 - 35; Hawkins et al., Nucl. Acid Res. 2004, 32, e62). Beide Vorgehensweisen sind jedoch stark sequenzabhängig und benötigen zusätzliche Informationen zum Aufbau einer solchen Sonde.

Häufig werden auch einfach markierte Sonden eingesetzt, wenn die entstehenden DNA-Fragmente über Fluoreszenzpolarisation nachgewiesen werden. Dabei wird ausgenutzt, dass sich durch den Einbau des Primers in den Duplexstrang die Rotationseigenschaften und damit die Fluoreszenzpolarisation des vergrößerten Moleküles ändern. Zum Teil werden dafür auch noch Hilfsmoleküle wie poly-Lysin zur Vergrößerung der Amplifikate (bsp. bei LNA-Primern) dem PCR-Ansatz zugesetzt (Gibson et al., Clin Chem. 1997, 43, 1336 - 41 und Simeonov et al., Nucl. Acid Res. 2002, 30, e91). Bei diesem Verfahren sind die Farbstoffe zwar kovalent gebunden, jedoch können die Farbstoffe nicht interkalieren. Die Messung der Fluoreszenzpolarisation ist aufwendiger als die Messung der Fluoreszenz.

Bei dem unspezifischen Nachweis der Amplifikation kann auch ein doppelstrang-bindender Farbstoff der PCR-Reaktion zugesetzt werden, der bei Interkalation an den Duplexstrang fluoresziert. Dabei wird über die Interkalation des Farbstoffes an den Duplexstrang eine Vermehrung der DNA in der Reaktion nachgewiesen. Dies geschieht jedoch völlig unspezifisch (SYBR^{®}-Green, Ethidiumbromid) und eine Unterscheidung zwischen erwünschten Amplifikaten und unspezifischen Amplifikaten ist ausgeschlossen (z.B. US 6,346,386; Zipper et al., Nucl. Acid Res. 2004, 32, e103; Poddar et al., J Clin Anal. 2004, 18, 265 - 70). Es ist ein großes Problem beim bisherigen Nachweis der Amplifikate über Fluoreszenz oder Fluoreszenzpolarisation, dass über die Spezifität der amplifizierten Sequenzen durch die einfache Quantifizierung der Fluoreszenz keine Aussage gemacht werden kann.

Durch die erfindungsgemäße Kombination eines doppelstrang-bindenden Farbstoffes (Fluoreszenzinterkalator), der seine Fluoreszenzeigenschaften mit der gezielten Bindung (Interkalation) an einen Doppelstrang ändert und einer sequenzabhängigen Hybridisierung lassen sich diese Probleme auf überraschende Weise umgehen. Dabei wird eine spezifische Nukleinsäuresonde mit einem doppelstrang-bindenden Farbstoff kovalent markiert. Durch die Bindung der spezifischen Sonde an die Ziel-Nukleinsäure kommt es zu einer sequenzspezifischen Ausbildung eines Doppelstranges, dies führt dann zu einer Zunahme der detektierbaren Fluoreszenz durch den doppelstrang-bindenden Farbstoff (Interkalator). Durch das Verfahren wird eine genauere Quantifizierung der Ziel-Nukleinsäuren möglich, da diese nicht von der Länge der Ziel-Nukleinsäuren abhängig ist. Wird hingegen ein freier, doppelstrang-bindender Farbstoff wie SYBR^{®}-Green verwendet, so kann umso mehr Farbstoff an den Doppelstrang binden, je länger dieser ist. Dadurch kommt es zu einer Zunahme der detektierbaren Fluoreszenz und die Menge an Ziel-Nukleinsäure kann nicht mehr genau bestimmt werden.

Ein weiterer großer Vorteil der Erfindung ist die Möglichkeit, ein Gemisch aus Nukleinsäuresonden einsetzen zu können, wobei z.B. die Nukleinsäuresonden mit unterschiedlichen Fluoreszenzinterkalatoren markiert sind. Dadurch wird es möglich, die verschiedenen Nukleinsäuresonden bei unterschiedlichen Fluoreszenzwellenlängen zu detektieren. Bei diesem Verfahren können dann gleichzeitig verschiedene Nukleinsäureproben amplifiziert und einzeln detektiert werden.

Ein anderes, PCR basiertes Verfahren zur Detektion und Quantifizierung nutzt die so genannte LUX^{®}-Primer Technologie (Nazarenko et al., Nucl. Acid Res. 2000, 30, e37 und Nazarenko et al., Nucl. Acid Res. 2000, 9, 2089-2195; Chen et al., J Virol Methods, 2004, 122, 57-61). Bei diesem Verfahren kommen für eine PCR-basierte Detektion zwei Nukleinsäurestränge (Außenprimer in einer PCR) zum Einsatz. Das Verfahren der vorliegenden Erfindung zeigt gegenüber diesem Verfahren den Vorteil, dass keine spezifischen Sequenzanforderungen an die einzusetzende Nukleinsäuresonde gestellt werden, um die Ausgangsfluoreszenz, die gleichzeitig die Basislinie innerhalb der real-time PCR definiert, zu reduzieren. Eine solche Reduktion der Basislinie ist entscheidend, um den Fluoreszenzanstieg überhaupt detektieren zu können. Um dieses zu erreichen, wird die Nukleinsäure in der LUX^{®}-Primer Technologie sekundärstrukturbehaftet aufgebaut (Sonden-Design), d.h. die gesamte Sequenz trägt z.B. terminal komplementäre Sequenzabschnitte, die unter nicht-hybridisierten Bedingungen eine Haarnadelstruktur ausbilden. Eine solche Haarnadelstruktur führt durch spezielle Interaktionen des im terminalen Bereich angebrachten Fluorophors zu einer Herabsetzung der Fluoreszenz des ungebundenen Moleküls. Die Auswahl einiger solcher Sequenzen ist bei Nazarenko (Nazarenko et al., Nucl. Acid Res. 2000, 30, e37 und Nazarenko et al., Nucl. Acid Res. 2000, 9, 2089-2195) für den Fall der LUX^{®} Sonden beschrieben.

Das erfindungsgemäße Verfahren kommt ohne die Entwicklung und den Aufbau solcher Haarnadelstrukturen aus. Erfindungsgemäß werden beispielsweise Außenprimer-Sequenzen verwendet, die ebenfalls für eine spezifische Amplifikation eingesetzt werden. Des Weiteren kann die erfindungsgemäße Nukleinsäuresonde auch als nicht verlängerbare Sonde in einer 3-Primer-Technik verwendet werden. Man erhält die hier vorgestellten Nukleinsäuresonden indem man im inneren Bereich der Nukleinsäuresequenz eine Basenposition durch eine homologe Base ersetzt, die zusätzlich über einen Linker verfügt, an welchem sich Farbstoffe, oder andere Moleküle kovalent anfügen lassen. Diese Nukleinsäuresonden können dann als Primer in PCR-Reaktionen eingesetzt werden. Einschränkungen hinsichtlich Polymerasen entfallen ebenfalls in dieser Technologie (vgl. auch TaqMan PCR, unten).

Bei der TaqMan Technologie, die von Lee et al. 1993 erstmals beschrieben wurde (Lee et al., Nucl. Acid Res. 1993, 16, 3761-3766), werden insgesamt drei Primer für eine Detektion bzw. Quantifizierung verwendet. Zwei Außenprimer dienen der Amplifikation der Zielsequenz. Der dritte Primer dient als doppelt-markierte Sonde. Bei den Markierungen handelt es sich um ein Fluorophor, üblicherweise am 5' Ende des Nukleinsäurestranges (z.B. FAM) und einen kompatiblen Quencher, üblicherweise am 3' Ende des Nukleinsäurestranges (z.B. TAMRA). Die so aufgebaute Nukleinsäuresonde verfügt über eine durch Fluoreszens-Resonanzenergie-Transfer (FRET) reduzierte Fluoreszens. Im Gegensatz zur vorliegenden Erfindung entsteht die Fluoreszenz des Fluorophors nicht durch eine Wechselwirkung des Farbstoffs mit dem Doppelstrang. Der Bindungsbereich der Sonde liegt zwischen den beiden Außenprimern. Die Tm-Werte (Schmelztemperaturwerte) der Primer sind dabei so gewählt (Sonden-Design), dass bei einem Temperaturgradienten nach unten zunächst die Sonde an einen Einzelstrang bindet und daraufhin die terminalen Primer. Die üblicherweise verwendete Taq-Polymerase verlängert daraufhin den zur Sonde stromaufwärts gelegenen Außenprimer. Trifft das Enzym auf die hybridisierte Sonde, wird mit Hilfe der 5'→3' Exonukleaseaktivität der Polymerase der 5' befindliche Bereich der Sonde abgespalten. Hierdurch wird der Resonanzenergietransfer von dem Fluorophor zum Quencher unterbrochen, wodurch die Fluoreszenz innerhalb des Reaktionsansatzes zunimmt. Polymerasen die nicht über diese Exonukleaseaktivität verfügen, können für eine TaqMan PCR nicht eingesetzt werden. Zudem verfügt die Sonde über eine 3' Phosphatgruppe. Diese dient dazu die Sonde als nicht verlängerbaren Primer fungieren zu lassen, was zur Folge haben würde, dass ein verkürztes Amplifikat zwischen Sonde und dem stromabwärtsgelegenen Primer entstünde, ohne Hydrolyse der Sonde (Nebenreaktion).

Die hier vorgestellte Technologie ist von vielen dieser Einschränkungen frei und kann daher wesentlich einfacher eingesetzt werden.

Auch unterscheidet sich die hier vorgestellte Technologie ganz erheblich von der sogenannten Molecular Beacon Technologie (Vet und Marras; Methods Mol Biol. 2005, 288, 273-90, Tyagi und Kramer; Nat. Biotechnol. 1996, 14, 303-308). Bei den sogenannten Molecular Beacons handelt es sich um strukturbehaftete Sonden, bei welchen ohne die Bindung an eine komplementäre Zielsequenz die terminalen Bereiche untereinander Hybridisieren. Diese Hybridisierung führt auch zu einer räumlichen Annäherung eines Fluorophors (z.B. im 5' Bereich) an einen Quencher (z.B. im 3' Bereich), wodurch die Ausgangsfluoreszenz des Fluorophors stark reduziert wird (FRET). Der Kopfbereich eines solchen Beacons ist dabei so gewählt, dass er komplementär ist zu der Zielsequenz, mit welcher die Sonde hybridisieren kann. Eine solche Hybridisierung führt auch zwangsläufig zu einer räumlichen Trennung der beiden terminalen Bereiche, wodurch sich das Quenching-Verhalten ändert bzw. die Fluoreszenz deutlich zunimmt.

Eine Kombination aus Beacon Technologie und LUX^{®} Technologie stellen die sogenannten Scorpion-Primer dar. Auch dieser Sondentyp verfügt über eine interne Haarnadelstruktur, ein Fluorophor sowie ein Quenchermolekül. Die sehr aufwendige Konstruktion solcher Sonden erfolgt erneut über Design-Programme.

Svanvik et al. verwenden peptidische Nukleinsäuren, die mit Cyaninfarbstoffen markiert sind, zur Detektion von Ziel-DNA (Svanvik et al. Anal. Biochem. 2000, 281, 26 - 35; Svanvik et al., Anal. Biochem. 2000, 287, 179 - 182 und US 6,329,144). Hierbei wird der Farbstoff endständig an die Sonde angebracht. Ein Nachteil der peptidischen Nukleinsäuren ist, dass sie nicht als Primer in einem PCR-Verfahren eingesetzt werden können, da sie von der Polymerase nicht verlängert werden können. Ein weiterer Vorteil gegenüber den bisher von Svanvik et al. beschriebenen PNA (Peptide Nucleic Acid)-Sonden liegt in dem Einsatz von handelsüblichen Synthesechemikalien für die Synthese der erfindungsgemäßen Nukleinsäuresonden. Für die Synthese der beschriebenen PNA-basierten Sonden werden spezielle und sehr teure Chemikalien benötigt, gleichzeitig kann der dort beschriebene Farbstoff nur in Verbindung mit PNA-basierten Sonden zum Einsatz kommen (Ladungsproblem des Farbstoffes). Bei dem hier beschriebenen Verfahren ist die Möglichkeit gegeben, die Farbstoffmarkierung mittels handelsüblicher Synthesechemikalien an einer beliebigen Stelle innerhalb des Sondenmoleküles, und nicht nur endständig, anzubringen. Des Weiteren stehen verschiedene Farbstoffe mit unterschiedlichen Emissionsspektren zur Verfügung die eine breit gestreute Anwendung ermöglichen.

Die von Svanvik et al. beschriebenen PNA-Sonden weisen im freien Zustand eine höhere Fluoreszenz auf als der freie Farbstoff, der für die Sonde verwendet wurde (Anal. Biochem. 2000, 281, Seite 32, rechte Spalte). Dies wird auf eine Wechselwirkung des Farbstoffes mit der PNA-Base zurückgeführt. Dadurch wird die Basislinie der Fluoreszenz nachteilig erhöht. Dieses Problem tritt bei den erfindungsgemäßen Nukleinsäuresonden nicht auf.

Die Verwendung der erfindungsgemäßen Nukleinsäuresonde mit kovalent gebundenem interkalierendem Farbstoff führt dazu, dass eine messbare Fluoreszenz erst entsteht, wenn die Nukleinsäuresonde zu einem Doppelstrang komplettiert ist, bzw. wenn die Nukleinsäuresonde als Doppelstrang vorliegt.

Durch die kovalente Bindung des Fluoreszenzfarbstoffes ergibt sich auch ein entscheidender Vorteil für die Anwendung in der Chiptechnologie (Biochip). Bei der Immobilisierung von erfindungsgemäßen Nukleinsäuresonden auf festen Trägern wie Biochips, bleibt der Farbstoff immer an der Nukleinsäuresonde gebunden. Im Gegensatz dazu muss bei der Verwendung von giftigem Ethidiumbromid der Farbstoff während des Verfahrens zugegeben werden und befindet sich dann frei in Lösung. Bei einem Waschvorgang des Chips befindet sich ebenfalls Ethidiumbromid in Lösung. Dadurch wird einerseits die Entsorgung der Chemikalien erschwert und andererseits steigt die Gefahr und die Belastung für die Menschen im Labor.

Svanvik et al. berichten, dass die Fluoreszenzwellenlänge bzw. die Wellenlänge des Absorptionsmaximums der PNA-Sonden sequenzabhängig ist. Des Weiteren ist auch die Amplitude bzw. die Signalstärke der Fluoreszenz frequenzabhängig. Dadurch muss einerseits für jede PNA-Sonde einzeln zunächst eine geeignete Detektionswellenlänge bestimmt werden und andererseits ist auch eine Quantifizierung der Nukleinsäureprobe erst nach Eichung jeder PNA-Sonde, die eine neue Sequenz aufweist, möglich ( Anal. Biochem. 2000, 281, S. 29 rechte Spalte und S. 30 linke Spalte). Diese Nachteile werden durch die erfindungsgemäßen Nukleinsäuresonden behoben.

Des Weiteren können die von Svanvik et al. vorgestellten PNA-Sonden auch nicht in einem PCR-Verfahren verlängert oder eingebaut werden, sondern arbeiten immer nach der 3 Primertechnik, wobei die PNA-Sonde nur an das Amplifikat bindet, aber nicht in dieses eingebaut wird.

Ein weiterer Vorteil der erfindungsgemäßen Nukleinsäuresonden liegt darin, dass beliebige Basenpositionen der Nukleinsäuresonde durch eine homologe Base ersetzt werden können, an die ein doppelstrang-bindender Fluoreszenzfarbstoff kovalent über eine Linkereinheit gebunden ist. Dadurch werden multiple Markierungen der Nukleinsäuresonden möglich. Werden zudem ausschließlich nicht endständige Basenpositionen mit einem Farbstoff markiert, so können die endständigen Basenpositionen für weitere Anwendungen genutzt werden. Beispielsweise können die endständigen Basen eine Affinitätsmarkierung aufweisen. So können Antigene, Antikörper, Proteine, Glykoproteine, Lecitine, Enzyme oder Coenzyme an die 3'- oder 5'- Enden kovalent gebunden werden, um eine biospezifische Wechselwirkung zu einem Bindungspartner zu erzeugen. Falls die endständigen Basenpositionen nicht verändert werden, können beispielsweise die PCR-Fragmente kloniert oder die entstandenen Fragmente sequenziert werden, da die natürliche Basenpaarung noch vorliegt. Ein weiterer Vorteil einer nicht endständig farbstoffmarkierten Nukleinsäuresonde liegt darin, dass die Hybridisierung an den Endbereichen des doppelsträngigen Hybrids schwächer ist und ein endständig gebundener Farbstoff schlechter interkalieren würde (z.B. an einem "freien" Ende, bei dem die endständigen Basenpositionen nicht hybridisiert, sondern einzelsträngig vorliegen, sodass der Farbstoff nicht in einen Doppelstrang interkalieren kann). Durch die nicht endständig markierten Nukleinsäuresonden kann daher die Genauigkeit der Verfahren, z.B. Quantifizierung von Nukleinsäureproben oder Basenfehlpaarungen, erhöht werden. Des Weiteren kann die erfindungsgemäße Nukleinsäuresonde dadurch als PCR-Primer verwendet werden, ohne dass die endständige Farbstoffmarkierung die PCR-Polymerase behindert, oder sogar blockiert.

Ein weiterer Vorteil der erfindungsgemäßen Nukleinsäuresonden ist darin zu sehen, dass die Farbstoffmarkierung nach der Synthese der Nukleinsäure vorgenommen werden kann. Die Farbstoffe müssen daher nicht schon während des Synthesevorgangs des DNA-Strangs eingeführt werden und daher muss nicht auf eine eventuelle chemische Veränderung der Farbstoffe während der DNA-Strang-Synthese geachtet werden.

Außerdem erlaubt es die hier vorgestellte Technik Mutationen im Bindungsbereich des markierten Nukleinsäurestranges aufzulösen. Je nach "Passgenauigkeit" bzw. der vorhandenen "missmatch"-Bindungen lassen sich zeitlich versetzt unterschiedlich hohe Fluoreszenzen detektieren.

Die vorliegende Erfindung betrifft Nukleinsäuresonden, ausgewählt aus der Gruppe bestehend aus DNA, RNA, LNA, CNA, HNA, ANA, FANA und Chimären aus diesen; wobei die Nukleinsäuresonden weitere Modifikationen am Zucker, an der Base oder am Phosphatrückgrat aufweisen können, und wobei eine oder mehrere nicht endständige Basenpositionen der Nukleinsäuresonden durch eine homologe Base ersetzt sind, an die ein doppelstrang-bindender Fluoreszenzfarbstoff kovalent über eine Linkereinheit gebunden ist.

In einer bevorzugten Ausführungsform ist zusätzlich mindestens ein Fluorophor kovalent an die Nukleinsäuresonde gebunden.

In einer weiteren Ausführungsform der Erfindung sind 2-10, weiter bevorzugt 3-10 und noch weiter bevorzugt 2-4 Basenpositionen der Nukleinsäuresonden durch eine homologe Base ersetzt, an die ein doppelstrang-bindender Fluoreszenzfarbstoff über eine Linkereinheit kovalent gebunden ist.

Bevorzugt werden Nukleinsäuresonden verwendet, wobei die Nukleinsäuresonden aus Chimären von DNA, RNA, LNA, CNA, HNA, ANA und FANA bestehen.

In einer weiter bevorzugten Ausführungsform der Nukleinsäuresonden besitzen die Nukleinsäuresonden kein peptidisches Rückgrat.

Noch weiter bevorzugt sind Nukleinsäuresonden, wobei die Fluoreszenzwellenlänge und die Signalstärke der farbstoffmarkierten Nukleinsäuresonden nicht von der Basensequenz oder der Länge der Nukleinsäuresonden abhängig ist.

In einer noch weiter bevorzugten Ausführungsform der Nukleinsäuresonden weisen die 3'-oder 5'-Enden der Nukleinsäuresonden eine Affinitätsmarkierung auf.

Weiter bevorzugt sind Nukleinsäuresonden, worin die Affinitätsmarkierung ein kovalent gebundenes Biomolekül darstellt, ausgewählt aus der Gruppe bestehend aus Antigenen, Antikörper, Proteinen, Lecitinen, Enzymen und Coenzymen.

Bevorzugt sind Nukleinsäuresonden, wobei die Nukleinsäuresonden mit Fluoreszenzfarbstoffen markiert sind, ausgewählt aus der Gruppe bestehend aus SYBR^{®} 101, SYBR^{®} 102, SYBR^{®} 103, Pyren-Derivaten, Ethidiumbromid-Derivaten oder Acridin-Derivaten.

Noch weiter bevorzugt sind Nukleinsäuresonden, wobei die Fluoreszenzfarbstoffe ausgewählt sind aus der Gruppe bestehend aus SYBR^{®} 101, SYBR^{®} 102 und SYBR^{®} 103.

In einer besonders bevorzugten Ausführungsform wird ein Gemisch aus mindestens 2 Nukleinsäuresonden verwendet, wobei mindestens eine Nukleinsäuresonde eine erfindungemäße Nukleinsäuresonde darstellt. Am meisten bevorzugt ist ein Gemisch aus 2 Nukleinsäuresonden, wobei mindestens eine Nukleinsäuresonde eine erfindungsgemäße Nukleinsäuresonde ist, noch bevorzugter stellen beiden Nukleinsäuresonden eine erfindungemäße Nukleinsäuresonde dar.

Des Weiteren betrifft die vorliegende Erfindung ein Verfahren zur Hybridisierung einer Nukleinsäuresonde mit einer Nukleinsäureprobe, umfassend die Schritte von:
a) dem Bereitstellen einer erfindungsgemäßen Nukleinsäuresonde,
b) dem in Kontaktbringen der Nukleinsäureprobe mit der Nukleinsäuresonde zur Hybridisierung der Nukleinsäureprobe mit der Nukleinsäuresonde,
c) dem Messen der Änderung des Fluoreszenzverhaltens.

Bevorzugt ist ein Verfahren, worin Nukleinsäureproben verwendet werden, ausgewählt aus der Gruppe, bestehend aus DNA, RNA, LNA und PNA.

Weiter bevorzugt ist ein Verfahren, worin die Nukleinsäureproben ausgewählt sind aus der Gruppe, bestehend aus DNA, RNA, LNA, CNA, HNA , ANA und FANA .

Eine weiter bevorzugte Ausführungsform der Erfindung ist ein Verfahren, worin die Nukleinsäureproben ausgewählt sind aus der Gruppe, bestehend aus DNA und RNA.

Bevorzugt ist ein Verfahren, worin verschiedene Farbstoffe bei unterschiedlichen Fluoreszenzwellenlängen detektiert werden können.

Des Weiteren ist ein Verfahren bevorzugt, worin verschiedene Nukleinsäuresonden bei unterschiedlichen Fluoreszenzwellenlängen detektiert werden können.

In einer bevorzugten Ausführungsform des Verfahrens erfolgt die Hybridisierung der Nukleinsäuresonde mit der Nukleinsäureprobe in einem PCR (Polymerase-Ketten-Reaktion)-Verfahren zur Amplifikation der Nukleinsäureprobe.

Weiter bevorzugt ist ein Verfahren, worin das Verfahren zur Amplifikation der Nukleinsäureproben ein Real-Time-PCR (Polymerase-Ketten-Reaktion) - Verfahren ist.

In einer noch weiter bevorzugten Ausführungsform des Verfahrens wird die Nukleinsäuresonde als nicht verlängerbarer Innenprimer verwendet.

In einem anderen bevorzugten Verfahren wird die Nukleinsäuresonde als Außenprimer verwendet.

Bevorzugt ist weiterhin ein Verfahren, worin die Änderung des Fluoreszenzverhaltens in Abhängigkeit der Zahl der Amplifizierungschritte bestimmt wird.

Weiter bevorzugt ist ein Verfahren, worin mindestens 2 Außenprimer-Paare verwendet werden, wobei mindestens ein Primer des Außenprimer-Paares eine farbstoffmarkierte Nukleinsäuresonden ist, und wobei die Außenprimer-Paare jeweils unterschiedliche Farbstoffe aufweisen oder das Fluoreszenzverhalten der Nukleinsäuresonden unterschiedlich ist, obwohl die gleichen Farbstoffe verwendet wurden. Bevorzugt wird das Fluoreszenzverhalten durch das Anbringen von zusätzlichen Fluorophoren verändert.

Noch weiter bevorzugt ist ein Verfahren, worin die Detektion der Hybridisierung der Nukleinsäuresonde mit der Nukleinsäureprobe durch Messung der Fluoreszenz oder der Fluoreszenzpolarisation erfolgt.

In den erfindungsgemäßen Verfahren werden bevorzugt mindestens zwei farbstoffmarkierte Nukleinsäuresonden in dem Verfahren verwendet.

In einer anderen bevorzugten Ausführungsform des Verfahrens wird die Hybridisierung der Nukleinsäuresonde mit der Nukleinsäureprobe zur direkten Detektion von Nukleinsäureproben verwendet.

In einer noch bevorzugteren Ausführungsform des Verfahrens wird ein Gemisch aus verschiedenen Nukleinsäuresonden verwendet, die mit verschiedenen Farbstoffen markiert sind oder sich das Fluoreszenzverhalten der Nukleinsäuresonden bei gleichem Farbstoff unterscheidet, und worin ein Gemisch aus Nukleinsäureproben verwendet wird, sodass je eine spezifische Nukleinsäuresonde mit je einer spezifischen Nukleinsäureprobe hybridisieren kann und wobei weitere unspezifische Nukleinsäureproben in dem Gemisch enthalten sein können. Bevorzugt unterscheidet sich das Fluoreszenzverhalten der Nukleinsäuresonden mit gleichen Fluoreszenzfarbstoffen dadurch, dass die Nukleinsäuresonden zusätzlich zu den interkalierenden Fluoreszenzfarbstoffen noch Fluorophore aufweisen. Die Detektion der unterschiedlichen Hybride aus Nukleinsäuresonde und Nukleinsäureprobe erfolgt hierbei bevorzugt bei unterschiedlichen Fluoreszenzwellenlängen.

In einer bevorzugten Ausführungsform der Erfindung wird ein Gemisch aus mindestens 2 Außenprimer-Paaren verwendet, worin jedes Außenprimer-Paar mindestens eine farbstoffmarkierte Nukleinsäuresonde umfasst, ausgewählt aus der Gruppe bestehend aus DNA, RNA, LNA, CNA, HNA, ANA und FANA und Chimären aus diesen, wobei die Nukleinsäuresonden weitere Modifikationen am Zucker, an der Base oder am Phosphatrückgrat aufweisen können, und wobei eine oder mehrere Basenpositionen der Nukleinsäuresonde durch eine homologe Base ersetzt wurden, an die ein doppelstrang-bindender Fluoreszenzfarbstoff kovalent über eine Linkereinheit gebunden ist, und wobei die Außenprimer-Paare jeweils unterschiedliche Farbstoffe aufweisen. Bevorzugt erfolgt die Markierung der Basen in dieser Ausführungsform nicht endständig.

Noch weiter bevorzugt ist ein Verfahren, worin die Detektion der Nukleinsäureproben auf Chips oder Partikeln (Beads) erfolgt.

In einer weiteren bevorzugten Ausführungsform des Verfahrens wird die Änderung des Fluoreszenzverhaltens in Abhängigkeit der Temperatur gemessen. Durch Temperaturveränderung wird eine Schmelzkurve erstellt und die Änderung des Fluoreszenzverhaltens lässt einen Rückschluss auf die Identität bzw. Fehler der Basenpaarungen zu.

Noch bevorzugter ist ein Verfahren, worin eine oder mehrere Nukleinsäuresonden auf einem festen Träger (Chip) immobilisiert sind. Dieses Verfahren kann verwendet werden, um die Nukleinsäureprobe zu Amplifizieren oder direkt zu Detektieren.

Bevorzugt wird bei dem Verfahren die Änderung des Fluoreszenzverhaltens in Abhängigkeit der Zahl der Amplifizierungschritte bestimmt.

Bei dem erfindungsgemäßen Verfahren erfolgt die spektroskopische Detektion eines Hybrids aus Nukleinsäuresonde und Nukleinsäureprobe bevorzugt bei einer Anregungswellenlänge in einem Bereich von 450 - 550 nm und einer Messwellenlänge von 480 - 580 nm.

Bevorzugt erfolgt die Detektion der Hybridisierung der Nukleinsäuresonde mit der Nukleinsäureprobe bei den erfindungsgemäßen Verfahren durch Messung der Fluoreszenz oder der Fluoreszenzpolarisation, besonders bevorzugt erfolgt die Detektion durch Messung der Fluoreszenz.

In einer weiter bevorzugten Ausführungsform der Verfahren, liegen die Nukleinsäureproben in einem Gemisch mit unspezifischen Nukleinsäuren vor, an die die Nukleinsäuresonden nicht binden können.

Die nachfolgend beschriebenen Nukleinsäuresonden können für alle erfindungsgemäßen Verfahren zur Hybridisierung von Nukleinsäuresonden an Nukleinsäureproben verwendet werden.
Die vorliegende Erfindung betrifft Nukleinsäuresonden aus DNA, RNA, LNA, CNA, HNA , ANA und FANA oder Chimären aus den aufgeführten Nukleinsäureanaloga und natürlich vorkommenden (DNA). Bevorzugt besteht die Nukleinsäuresonde aus RNA, LNA, CNA, und HNA. Besonders bevorzugt besteht die Nukleinsäuresonde aus Chimären von DNA, RNA, LNA, CNA, HNA, ANA und FANA. Falls die Nukleinsäuresonde aus Chimären der aufgeführten Nukleinsäureanaloga besteht, muss das 3'-Ende der Nukleinsäuresonde aus DNA bestehen, damit die Polymerasen während der PCR-Reaktion weitere Basen anknüpfen können.

Bevorzugt können die Nukleinsäuresonden zusätzlich Modifikationen am Zucker, an der Base oder am Phosphatrückgrat aufweisen.

Die Nukleinsäuresonden werden hergestellt, indem eine oder mehrere Basenpositionen durch eine homologe Base ersetzt werden, an die ein doppelstrang-bindender Fluoreszenzfarbstoff kovalent über eine Linkereinheit gebunden ist. Bei den hier verwendeten Markierungen handelt es sich um sogenannte Fluoreszenz-Interkalatoren, die nach Bindung an eine Zielsequenz ihre Fluoreszenzeigenschaften stark ändern. Die Detektion bzw. Diskriminierung der Zielsequenz/en kann durch Messen der Fluoreszenz oder der Fluoreszenzpolarisation erfolgen.

Jede Nukleinsäuresonde enthält 1-10 doppelstrang-bindende Fluoreszenzfarbstoffe, bevorzugt sind 2-10, noch bevorzugter sind 2-4 doppelstrang-bindende Fluoreszenzfarbstoffe, die jeweils über eine Linkereinheit kovalent an jeweils eine Base der Nukleinsäuresonde gebunden sind. Die Nukleinsäuresonde kann den gleichen Fluoreszenzfarbstoff oder verschiedene Fluoreszenzfarbstoffe an den farbstoffmarkierten Basen aufweisen. Zusätzlich kann sie bevorzugt Fluorophore enthalten. Besonders bevorzugt ist eine Nukleinsäuresonde, die einen kovalent gebundenen, interkalierenden Fluoreszenzfarbstoff und ein kovalent gebundenes Fluorophor aufweist.

Bevorzugte Farbstoffe für die kovalente Verknüpfung mit einer Linkereinheit sind SYBR^{®} 101, SYBR^{®} 102 oder SYBR^{®} 10, Pyren-Derivate, Ethidiumbromid-Derivate und AcridinDerivate. Noch bevorzugtere Farbstoffe sind SYBR^{®} 101, SYBR^{®} 102 oder SYBR^{®} 103. Besonders bevorzugt ist der Farbstoff SYBR^{®} 102.

Hierbei können die Basen an beliebigen Stellen der Nukleinsäuresonde mit Farbstoffen markiert werden. Bevorzugt ist es, nicht endständige Basenpositionen mit den Fluoreszenzfarbstoffen zu markieren, weiter bevorzugt ist es nicht die 2 äußersten Basenpositionen eines Nukleinsäureendes, noch bevorzugter nicht die 3 äußersten und am meisten bevorzugt nicht die 4 äußersten Basenpositionen eines Nukleinsäureendes zu markieren.

In einer weiteren bevorzugten Ausführungsform der Nukleinsäuresonde befinden sich die farbstoffmarkierten Basen in dem mittleren Abschnitt der Nukleinsäuresonde, wenn man die Nukleinsäuresonde/-basensequenz der Länge nach in 3 gleiche Teilabschnitte unterteilt.

Die Markierung erfolgt über den Einbau von modifizierten Basen, die eine Linkereinheit besitzen, an die anschließend der interkalierende Fluoreszenzfarbstoff kovalent gebunden werden kann. Die Synthese der Nukleinsäuresonde, die eine spezifische Sequenz und Basen mit Linkereinheiten an den gewünschten Basenpositionen aufweist, kann nach den üblichen Syntheseverfahren hergestellt werden. Bevorzugt wird die Nukleinsäuresonde nach der Phosphoramidit-Methode erzeugt, wenn es sich bei der Nukleinsäuresonde um DNA oder RNA handelt.

Zusätzlich werden bevorzugt ausschließlich nicht endständige Basenpositionen markiert. Dabei ist es besonders bevorzugt, dass die endständigen Basenpositionen für weitere Anwendungen genutzt werden können. Dazu können die endständigen Basen entweder natürliche Basen (Adenin, Guanin Cytosin oder Thymin) sein, oder eine Affinitätsmarkierung aufweisen. Bevorzugt weisen die Nukleinsäuresonden eine Affinitätsmarkierung auf.

Bei einer natürlichen Basenpaarung an den Enden der Nukleinsäuresonde ist es möglich, die unmodifizierten Enden für eine der PCR nachgeschaltete direkte Klonierung mit üblichen Verfahren zu verwenden. Dadurch können z.B. auch Splicingvarianten von Genen ermittelt werden.

Ein weiterer Vorteil der natürlichen Basenpaarung an den Enden der Nukleinsäuresonde entsteht dadurch, dass keine Pseudonukleotide eingesetzt werden und daher die amplifizierte Basenabfolge sequenziert werden kann. Werden die endständigen Basenpositionen nicht verändert, können beispielsweise die PCR-Fragmente kloniert oder die entstandenen Fragmenten sequenziert werden, da die natürliche Basenpaarung noch vorliegt. Beispielsweise kann eine reverse Transkriptions-PCR durchgeführt werden, wobei die amplifizierten Nukleinsäureproben kloniert und z.B. sequenziert, exprimiert etc. werden können.

Durch eine endständige Modifikation der Nukleinsäuresonden durch Affinitätsmarkierungen kann nach der Quantifizierung durch Real-Time-PCR, das erhaltene Templat z.B. direkt am Träger immobilisiert werden, um als Nukleinsäuresonde für weitere Tests zur Verfügung zu stehen. Oder es kann über diese Modifikation eine Aufreinigung stattfinden und die amplifizierte Sequenz steht für weitere Anwendungen zur Verfügung. Bevorzugte Affinitätsmarkierungen, die kovalent an das 3'- oder 5'- Enden angebracht werden können, um eine biospezifische Wechselwirkung zu einem Bindungspartner zu erzeugen, sind Antigene, Antikörper, Proteine, Lecitine, Enzyme oder Coenzyme.

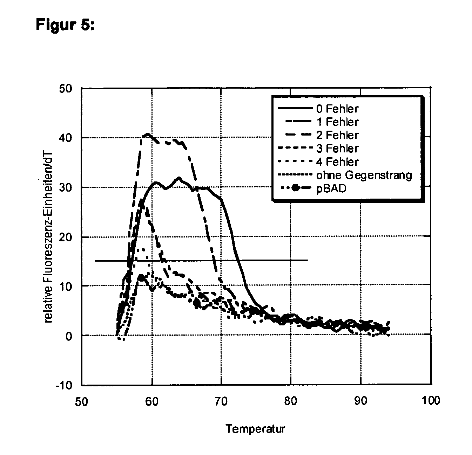

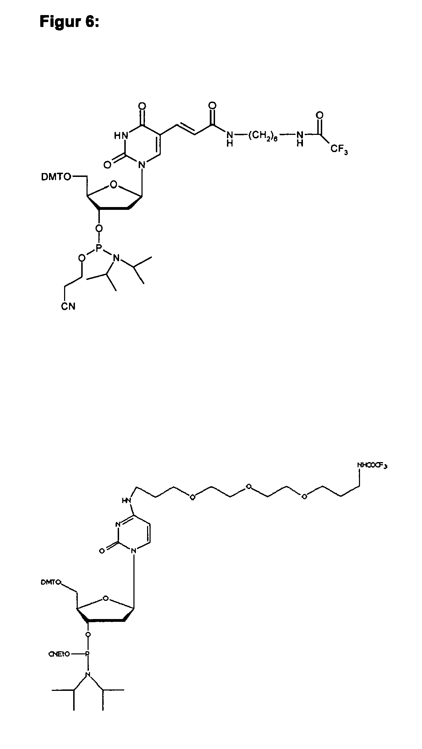

In den erfindungsgemäßen Nukleinsäuresonden sind einzelne Basenpositionen durch eine homologe Base ersetzt, die eine Linkereinheit aufweisen. Dabei wird die Linkereinheit so ausgewählt, dass sie jeweils an den spezifischen interkalierenden Farbstoff gebunden werden kann. Hierbei können übliche Basen mit Linkereinheiten verwendet werden. Bevorzugt weisen die Linkereinheiten Aminogruppen oder SH-Gruppen auf, wobei jene funktionellen Gruppen, die mit dem Farbstoff reagieren, bis zur Kopplung an den Farbstoff mit einer Schutzgruppe versehen sein können. Besonders bevorzugt wird eine Linkereinheit mit einer Aminogruppe verwendet. Bevorzugte Beispiele für modifizierte Basen mit Linkereinheiten (beispielsweise von der Firma Chemgenes Corp., USA erhältlich) sind der Amino Modifier dT oder der Cytidin N4 Aminolinker, die in Figur 6 dargestellt sind. Die Kopplung von SYBR-102 an die entschützte Aminogruppe der beiden modifizierten Basen kann z.B. nach der Anleitung des Herstellers von SYBR-102 erfolgen (Firma Invitrogen).

Linkereinheiten die eine endständige SH-Gruppe aufweisen, können beispielsweise über eine Maleimidaktivierung an Farbstoffe gekoppelt werden.

Die Linkereinheiten, die freie Aminogruppen aufweisen, können mit Imidoestern oder Isothiocyanaten oder mit reaktiven Succinimidylestern wie beispielsweise den SYBR^{®} 101-, SYBR^{®} 102- oder SYBR^{®} 103- Farbstoffen (die SYBR^{®}-Farbstoffe sind von der Firma Invitrogen erhältlich), reagieren.

In einer Ausführungsform der Nukleinsäuresonde, die für alle erfindungsgemäßen Verfahren verwendet werden kann, ist zusätzlich zu dem doppelstrang-bindenden Fluoreszenzfarbstoff (Reporterfarbstoff), ein weiteres Fluorophor kovalent an die Nukleinsäuresonde gebunden. Dieses Fluorophor beeinflusst das Fluoreszenzverhalten der Nukleinsäuresonde. Dadurch wird entweder die Messfrequenz des interkalierenden Farbstoffs verschoben oder es wird die Fluoreszenz des Reporterfarbstoffes im ungebundenen / nicht-interkalierten Zustand absorbiert, um die Grundfluoreszenz der Sonde noch weiter zu senken.

Diese Ausführungsform ermöglicht eine neue Variante des FRET-Systems, das einen Farbstoff und einen Quencher verwendet. Durch die Bindung des Reporterfarbstoffes im Doppelstrang, bei Bindung der Nukleinsäuresonde an die Nukleinsäureprobe (Zielsequenz), werden Reporterfarbstoff und Quencherfarbstoff räumlich von einander getrennt und beeinflussen sich gegenseitig nicht mehr. Als Quencher wird bevorzugt eine Dabcyl-Markierung eingesetzt, die sich in räumlicher Nähe des Reporterfarbstoffs befinden muss.

In einer anderen bevorzugten Ausführungsform wird ein Gemisch aus mindestens 2, noch bevorzugter mindestens 3 und am meisten bevorzugt aus mindestens 4 Nukleinsäuresonden verwendet, ausgewählt aus der Gruppe bestehend aus DNA, RNA, LNA, CNA, HNA, ANA und FANA; wobei die Nukleinsäuresonden weitere Modifikationen am Zucker, an der Base oder am Phosphatrückgrat aufweisen können, und wobei eine oder mehrere nicht endständige Basenpositionen der Nukleinsäuresonden durch eine homologe Base ersetzt wurden, an die ein doppelstrang-bindender Fluoreszenzfarbstoff kovalent über eine Linkereinheit gebunden ist. Ein Gemisch aus 2 Nukleinsäuresonden enthält die beiden Nukleinsäuresonden bevorzugt in einem Verhältnis von 1/10 bis 10/1, weiter bevorzugt von 1/3 bis 3/1. Das Gemisch aus Nukleinsäuresonden kann für alle nachfolgend beschriebenen Verfahren zur Hybridisierung von Nukleinsäuresonde und Nukleinsäureprobe eingesetzt werden. Bevorzugt können zusätzlich nicht erfindungsgemäße Nukleinsäuresonden in dem Gemisch enthalten sein. Besonders bevorzugt enthält dieses Gemisch mindestens eine erfindungsgemäße Nukleinsäuresonde.

Des Weiteren betrifft die vorliegende Erfindung ein Verfahren zur Hybridisierung von Nukleinsäuresonden mit Nukleinsäureproben, wobei alle markierten Nukleinsäuresonden bzw. alle beschriebenen Ausführungsformen der Nukleinsäuresonden verwendet werden können, die oben beschrieben worden sind. Dieses erfindungsgemäße Verfahren umfasst drei Verfahrensschritte, wobei diese Schritte in dem Verfahren mehrmals angewendet werden können und keine Reihenfolge der Schritte festgelegt ist. Das Verfahren kann auch um weitere Verfahrensschritte ergänzt werden.

Das Verfahren zur Hybridisierung von Nukleinsäuresonden mit Nukleinsäureproben enthält einen Schritt der Bereitstellung einer Nukleinsäuresonde. Diese Nukleinsäuresonde wurden im Vorangegangenen beschrieben und diese kann zusätzlich bevorzugt auch in einem Gemisch aus erfindungsgemäßen Nukleinsäuresonden verwendet werden, wobei dieses Gemisch auch Nukleinsäuresonden ohne Farbstoffmarkierung umfassen kann. Bevorzugt enthält dieses Gemisch auch Nukleinsäuresonden, die nicht erfindungsgemäß bzw. nicht mit einem Farbstoff markiert sind.

In dem Gemisch aus mindestens 2, noch bevorzugter mindestens 3 und am meisten bevorzugt aus mindestens 4 Nukleinsäuresonden werden bevorzugt Nukleinsäuresonden verwendet, die jeweils unterschiedliche Fluoreszenzeigenschaften aufweisen und deshalb auch in einem Gemisch selektiv detektiert werden können. Dabei können die unterschiedlichen Fluoreszenzeigenschaften durch unterschiedliche Fluoreszenzfarbstoffe, oder durch das Anbringen von zusätzlichen Fluorophoren erreicht werden. Fluorophore sind hierbei kovalent gebundene Farbstoffe, die nicht in einen Doppelstrang interkalieren können. Hierbei können alle bekannten Fluorophore verwendet werden. In einem bevorzugten Gemisch aus 2 unterschiedlichen Nukleinsäuresonden wird bevorzugt in beiden Nukleinsäuresonden der selbe Farbstoff verwendet, jedoch wird eine Nukleinsäuresonde zusätzlich mit einem Fluorophor z.B. TAMRA (Tetramethylrhodamin)-Fluorophor, versehen. Dadurch verschiebt sich die Messfrequenz der Nukleinsäuresonde und die beiden Nukleinsäuresonden werden unterscheidbar. Als Anregungswellenlänge dient die für den interkalierenden Fluroreszenzfarbstoff charakteristische Wellenlänge. Die Detektion der Nukleinsäuresonde ohne Fluorophor erfolgt dann bei der für den Farbstoff charakteristischen Fluoreszenzwellenlänge. Sind das Fluorophor (z.B. TAMRA) und der interkalierende Farbstoff in der anderen Nukleinsäuresonde räumlich nahe genug beieinander, so überträgt der interkalierende Farbstoff die eingestrahlte Energie (charakteristische Anregungswellenlänge des interkalierenden Farbstoffs) auf das Fluorophor. Das Fluorophor emittiert diese Energie dann bei einer für das Fluorophor charakteristischen Wellenlänge, die sich von der des interkalierenden Farbstoffs unterscheidet. So können bei Einstrahlung von Licht mit einer für den interkalierenden Farbstoff charakteristischen Anregungswellenlänge die beiden Nukleinsäuresonden gleichzeitig selektiv bei unterschiedlichen Wellenlängen detektiert werden.

Zusätzlich kann die Nukleinsäuresonde bevorzugt an einen festen Träger gebunden sein (immobilisiert). Bevorzugt wird die Nukleinsäuresonde hierbei auf einer Chipoberfläche (Biochip) direkt immobilisiert, oder im nachhinein, nach einer Probenhybridisierung (der Target oder Zielsequenz) zugegeben wird. Probenhybridisierung kann hier beispielsweise das Mischen der zu untersuchenden DNA/RNA-Mischung mit einer Test-DNA (z.B. Poly-T-Sequenz), die immobilisiert sein kann, bedeuten. in einem nachfolgenden Schritt kann dann eine farbstoffmarkierte Sonde zugefügt werden, so können mehrere Sonden nacheinander am selben Chip austestet werden. Ein Chip ist dabei ein fester Träger, beispielsweise aus Glas oder einem Polymer, an den z.B. DNA-Einzelstränge gebunden werden können. Analog dazu kann die Detektion von Nukleinsäureproben auch auf Partikeln (Beads) erfolgen. Hierbei können handelsübliche Partikel verwendet werden.

In einem weiteren Schritt wird die Nukleinsäuresonde mit der Nukleinsäureprobe in Kontakt gebracht. Bei diesem Schritt kann eine Amplifikation der Nukleinsäureprobe erfolgen, oder es wird nur die eingesetzte Menge an Nukleinsäureprobe detektiert. Die Nukleinsäuresonde kann mit der Nukleinsäureprobe zu einem Doppelstrang hybridisieren, woraufhin der interkalierende Fluoreszenzfarbstoff der Nukleinsäuresonde in den Doppelstrang interkaliert und sich dessen Fluoreszenzverhalten ändert.

In einem weiteren Schritt des Verfahrens wird die Änderung des Fluoreszenzverhaltens gemessen. Hierbei wird die Fluoreszenz oder die Fluoreszenzpolarisation mit üblichen Verfahren gemessen. Bevorzugterweise wird die Fluoreszenz gemessen. In Abhängigkeit der Konzentration an Hybrid aus Nukleinsäuresonde und Nukleinsäureprobe entstehen unterschiedlich hohe Fluoreszenzwerte. Die Messung der Fluoreszenz oder der Fluoreszenzpolarisation ist eine Endpunktbestimmung, wenn das Verfahren nach der Messung beendet ist, oder ist eine Zwischenmessung, wenn weitere Schritte durchgeführt werden (z.B. bei einem Real-Time-PCR-Verfahren). Bei der gleichzeitigen Verwendung von verschiedenen Nukleinsäuresonden mit unterschiedlichen Fluoreszenzfarbstoffen können die Hybride aus Nukleinsäuresonde und Nukleinsäureprobe jeweils selektiv bei der für den Fluoreszenzfarbstoff oder die Nukleinsäuresonde charakteristischen Fluoreszenzwellenlänge detektiert werden.

Falls im Rahmen einer Multiplexanwendung zwei unterschiedliche Fluoreszenzfarbstoffe bei zwei unterschiedlichen Nukleinsäuresonden eingesetzt werden sollen, ist es zusätzlich bevorzugt, anstelle der Verwendung von zwei unterschiedlichen Fluoreszenzfarbstoffen, eine Nukleinsäuresonde mit einem zweiten Fluorophor zu versehen. Dadurch kommt es zu einer Verschiebung des Messbereichs der Fluoreszenz dieser doppelt markierten Nukleinsäuresonde. So weisen die beiden Nukleinsäuresonden ein unterschiedliches Fluoreszenzverhalten auf und können in einem Gemisch vorliegen und selektiv detektiert werden. Bevorzugt ist die Verwendung eines TAMRA Fluorophors zusammen mit einem Interkalatorfarbstoff.

In einer bevorzugten Ausführungsform des Verfahrens enthält der Schritt des "In-Kontaktbringens" von Nukleinsäuresonde und Nukleinsäureprobe einen Amplifizierungsschritt. Das Verfahren kann dann als PCR- Verfahren bezeichnet werden, in dem die Nukleinsäureprobe amplifiziert wird. Bei diesem Verfahren kann eine spezifische Probennukleinsäuresequenz amplifiziert werden, wobei diese DNA-Zielsequenz bevorzugt in einer Mischung mit unspezifischen DNA-Sequenzen vorliegen kann. Die Bindung zwischen Nukleinsäuresonde und Zielsequenz findet nur statt, wenn ein bestimmter Bereich spezifisch amplifiziert wurde. Durch die spezifische Amplifizierung ist dann eine deutlich erhöhte bzw. veränderte Fluoreszenz messbar. In Abhängigkeit der eingesetzten Konzentration der farbstoffmarkierten Primers entstehen unterschiedliche hohe Fluoreszenzwerte, jedoch wird der Zeitpunkt der erstmaligen Fluoreszenz nur unmerklich verändert. Bevorzugt wird eine Fluoreszenzentwicklung ab dem 10. Zyklus, noch bevorzugter ab dem 7. Zyklus und am meisten bevorzugt ab dem 5. Zyklus des PCR-Verfahrens beobachtet, wobei der Term "Zyklus" hier einen Amplifikationsschritt eines PCR-Verfahrens bezeichnet.

Der Nachweis der erfindungsgemäßen Nukleinsäuresonde ist spezifischer als eine einfache Dotierung des PCR-Reaktionsansatzes mit SYBR^{®}-Green oder Ethidiumbromid, da diese Farbstoffe unspezifisch in alle Doppelstränge interkalieren. Durch die Wahl unterschiedlicher Farbstoffe an verschiedenen Nukleinsäuren lassen sich zudem mehrere Zielsequenzen parallel nachweisen. Bei der gleichzeitigen Verwendung von verschiedenen Nukleinsäuresonden mit unterschiedlichen Fluoreszenzfarbstoffen können die Hybride aus Nukleinsäuresonde und Nukleinsäureprobe jeweils selektiv bei der für den Fluoreszenzfarbstoff charakteristischen Fluoreszenzwellenlänge detektiert werden.

Die erfindungsgemäßen Nukleinsäuresonden können in diesem PCR- Verfahren als Primer verwendet werden. In einem PCR-Verfahren können zwei oder drei Primer verwendet werden (Zwei-Primer-Technik bzw. Drei-Primer-Technik). Wird ein Gemisch aus verschiedenen Außen-Primerpaaren verwendet, so können mehrere unterschiedliche erfindungsgemäße Nukleinsäuresonden verwendet werden. Zusätzlich zu der erfindungsgemäßen Nukleinsäuresonde können bevorzugt weitere Primer verwendet werden, die nicht erfindungsgemäß sind.

Bei der Drei-Primer-Technik sind zusätzlich Innenprimer vorhanden. Bei der Drei-Primer-Technik können die Innenprimer und/oder Außenprimer erfindungsgemäße Nukleinsäuresonden darstellen. Noch bevorzugter sind zwei erfindungsgemäße Nukleinsäuresonden in einem Außenprimer-Paar vorhanden.

Bevorzugt enthält das Gemisch aus Nukleinsäuresonden mindestens 2 verschiedene Außenprimer-Paare, weiter bevorzugt genau 2 verschiedene Außenprimer-Paare. Diese zwei Außenprimer-Paare enthalten mindestens eine erfindungsgemäße Nukleinsäuresonde.

Zusätzlich ist bevorzugt, dass die erfindungsgemäßen Nukleinsäuresonden der Außenprimer-Paare unterschiedliche Farbstoffe aufweisen oder sich die erfindungsgemäßen Nukleinsäuresonden bei gleichem Farbstoff dennoch (wie oben beschrieben) in ihrem Fluoreszenzverhalten unterscheiden. Dadurch wird eine selektive Detektion der unterschiedlichen Hybride aus Nukleinsäuresonde und Nukleinsäureprobe in einem Gemisch aus unterschiedlichen Hybriden, bei verschiedenen Fluoreszenzwellenlängen möglich. Dabei bindet jeweils eine spezifische Nukleinsäuresonde an die dazu komplementäre Probennukleinsäure. In diesem Gemisch können zusätzlich auch unspezifische Probennukleinsäuren vorliegen, an die keine der Nukleinsäuresonden binden kann.

In einer weiteren bevorzugten Ausführungsform der Erfindung wird ein Multiplex-Verfahren durchgeführt. Hierbei wird eine Nukleinsäuresonde verwendet, die einen doppelt markierten PCR-Primer darstellt, hierbei wird jedoch eine Fluoreszenzverschiebung mittels eines zweiten Fluorophors erreicht. Bevorzugt ist die Verwendung eines TAMRA Fluorophors zusammen mit einem Interkalatorfarbstoff.

In einer weiteren bevorzugten Ausführungsform der Erfindung wird ein Multiplex-Verfahren durchgeführt. Hierbei wird eine Nukleinsäuresonde verwendet, die einen doppelt markierten PCR-Primer darstellt, hierbei wird jedoch eine Fluoreszenzverschiebung mittels eines zweiten Fluorophors erreicht. Bevorzugt ist die Verwendung eines TAMRA Fluorophors zusammen mit einem Interkalatorfarbstoff.

In der Zwei-Primer-Technik sind zwei Außenprimer (Primer-Paar) notwendig, wobei einer der Außenprimer unmarkiert (Primer 1) und der andere Außenprimer eine erfindungsgemäße Nukleinsäuresonde (Primer 2) sein kann. Bevorzugt ist, dass beide Außenprimer eine erfindungsgemäße Nukleinsäuresonde darstellen.

Bei diesem System kann direkt der amplifizierte DNA-Sequenzbereich, welcher die Modifikationen an dem/den Endbereich/en des Amplifikats trägt, nachgewiesen werden. Dabei ändert sich die Fluoreszenz der Farbstoffsonde in Abhängigkeit von der Bindung/dem Einbau der Sonde an/in die Nukleinsäure.

Zusätzlich bevorzugt ist, dass bei der Zwei-Primer-Technik die Nukleinsäuresonde dazu eingesetzt wird, eine Zielsequenz und deren Häufigkeit (Quantität) z.B. in einer Mischung, oder in einer Probe zu detektieren. Die Fluoreszenzentstehung ist an die Templatmenge gebunden. Innerhalb einer bestimmten Zyklenzahl entsteht eine bestimmte Menge an fluoreszierenden DNA-Doppelsträngen, jedoch nur wenn die eingesetzten Primer spezifisch für dieses Templat konzipiert wurden. Bei diesem Verfahren wird die Fluoreszenz nach jedem Amplifikationszyklus gemessen. In einem ersten Schritt des Verfahrens werden zunächst die nötigen Amplifikationszyklen für mehrere bekannte Probenkonzentrationen/ - mengen für Eichzwecke bestimmt. Die Zahl der benötigten Amplifikationszyklen ergibt sich aus der Zahl der Zyklen die notwendig ist, um eine Mindestfluoreszenz (Fluoreszenzschwellenwert) zu erreichen. Anhand von einer Eichgeraden kann dann eine Konzentrationsbestimmung einer unbekannten Menge an Probennukleinsäure vorgenommen werden, indem auch bei der unbekannten Probennukleinsäure die Zahl der notwendigen Amplifikationszyklen bis zur detektierbaren Fluoreszenz bestimmt wird. Je höher die Templatkonzentration ist, desto früher entwickelt sich eine messbare Fluoreszenz. Bevorzugt ist, dass eine Erhöhung der Probenkonzentration/ -Menge um den Faktor 1000 zu einer Verringerung der benötigten Amplifikationszyklen um mindestens 10 Zyklen führt. Noch weiter bevorzugt ist, dass eine Erhöhung der Probenkonzentration/ - Menge um den Faktor 100 zu einer Verringerung der benötigten Amplifikationszyklen um mindestens 10 Zyklen führt.

In Beispiel 2 ist ein Fluoreszenzschwellenwert nach dem 8. Zyklus bei einer eingesetzten Menge von 10 ng Templat bzw. nach dem 20. Zyklus bei einer eingesetzten Menge von 0,01 ng Templat erreicht. Dies entspricht einer Differenz von 12 Zyklen bei einer Verringerung der Probenmenge um den Faktor 1000. Nur wenn die zu untersuchende Probennukleinsäure die Zielsequenz enthält, entsteht eine messbare Fluoreszenz innerhalb einer bestimmten Zyklenzahl. Es kann daher sowohl der Nachweis, als auch eine Quantifizierung einer Zielsequenz vorgenommen werden.

In der Drei-Primer-Technik (Primer Gruppe aus drei Primern) kann die erfindungsgemäße Nukleinsäuresonde als nicht-verlängerbare Sonde (Innenprimer, z.B. 3' durch Phospat blockiert) oder als Außenprimer eingesetzt werden. Die Hybridisierung des erfindungsgemäßen Innenprimers (Nukleinsäuresonde) mit der Nukleinsäureprobe führt dann zu einer Veränderung des Fluoreszenzverhaltens, die in dem Detektionsschritt gemessen wird.

In diesem Verfahren kann die Zunahme des zu amplifizierenden Teilstückes (Bereich zwischen den beiden Außenprimern), durch Bindung der Sonde, mitverfolgt werden (während der Annealing-Phase). In dem Drei-Primer-Verfahren ist entweder mindestens ein Außenprimer oder der Innenprimer eine erfindungsgemäße Nukleinsäuresonde. Besonders bevorzugt ist mindestens ein Außenprimer-Paar und der Innenprimer eine erfindungsgemäße Nukleinsäuresonde. Die als nicht-verlängerbarer Innenprimer fungierende Nukleinsäuresonde bindet während der Annealingphase der PCR-Reaktion an die einzelsträngige Zielsequenz. Die durch Interkalation veränderte Fluoreszenz lässt sich hierbei zur Quantifizierung nutzen. Durch die Spezifität der farbstoffmarkierten Sonde wird sichergestellt, das nur spezifische Amplifikate nachgewiesen werden. Es besteht so nicht die Gefahr, dass unspezifisch amplifizierte Sequenzen mit detektiert werden können.

In einer weiteren Ausführungsform der Erfindung wird eine Nukleinsäuresonde verwendet, die einen doppelt markierten PCR-Primer darstellt, wie bereits bei den Ausführungsformen der Nukleinsäuresonden beschrieben. Die Nukleinsäuresonde trägt in diesem Verfahren zusätzlich zu dem Farbstoff (Reporterfarbstoff) einen zweiten Farbstoff, der in der Lage ist, die Fluoreszenz des Reporterfarbstoffs zu unterdrücken, solange der Reporterfarbstoff nicht interkaliert. Erst nach Bindung der Nukleinsäuresonde an eine Probennukleinsäure (Hybridisierung) tritt Fluoreszenz auf, da durch eine Konformationsänderung die Wechselwirkung zwischen dem Reporterfarbstoff und dem Quencherfarbstoff aufgehoben wird. Durch dieses Verfahren wird die Anfangsfluoreszenz durch den Quencherfarbstoff vor Einbau in das Amplifikat weiter reduziert.

In einer weiteren bevorzugten Ausführungsform des Verfahrens wird die erfindungsgemäße Nukleinsäuresonde zum direkten Nachweis von einzelsträngigen Nukleinsäureproben, deren Derivaten oder Analoga (z.B. RNA, LNA, PNA, ANA, FANA ect.), ohne deren Amplifikation eingesetzt, wobei durch die sequenz-spezifische Bindung/Einbau eine Fluoreszenzänderung (Absorptions-, Emissions- und Intensitätsänderung) erzeugt wird, die gemessen werden kann. Bevorzugt wird das Verfahren zum direkten Nachweis von natürlich vorkommenden Nukleinsäureproben in der medizinischen Diagnostik verwendet.

Hierbei können zusätzlich bevorzugt Mutationen über Schmelzpunktskurven der Duplexe aus Nukleinsäuresonde und nachzuweisender Nukleinsäure detektiert werden. Dabei ändert sich die detektierbare Fluoreszenz in Abhängigkeit der Schmelztemperatur der hybridisierten Proben. Damit ist es möglich festzustellen, ob die Nukleinsäuresonde als Einzelstrang (keine Fluoreszenz) oder Doppelstrang vorliegt. Mit steigender Anzahl an Fehlern bzw. Abweichungen zwischen Nukleinsäuresonde und Nukleinsäureprobe sinkt die Schmelztemperatur der entsprechenden Doppelstränge. Je geringer die Anzahl der Fehlpaarungen ist, desto stärker müssen die Doppelstränge aufgeheizt werden, um eine Abnahme der Fluoreszenz zu erreichen. Man kann auf diese Art und Weise über die Temperatur (oder einen zu definierenden Schwellenwert) Rückschlüsse auf die Passgenauigkeit der beiden DNA-Stränge ziehen. Dadurch kann die Nukleinsäuresonde zum Nachweis von Mutationen / Polymorphismen innerhalb des Bindungsbereiches eingesetzt werden (Ein-Primer-Technik). Die Bindungsaffinität der Nukleinsäuresonde an die untersuchte Sequenz nimmt mit steigender Unterschiedlichkeit zwischen Nukleinsäuresonde und Nukleinsäureprobe stark ab, so dass über die zeitliche Messung der Fluoreszenz des Doppelstranges (Temperatur- oder Denaturierungsgradient) der Schmelzpunkt und somit die "Passgenauigkeit" ermittelt werden kann. Hierdurch wird eine Unterscheidung zwischen verschiedenen DNA-Typen / Punktmutationen möglich.

In einer Ausführungsform wird das Verfahren zur Diskriminierung von Allelen verwendet. Durch das erfindungsgemäße Verfahren wird es möglich, zunächst festzustellen, ob eine Mutation vorliegt. Hierbei kann eine Tm-Wertbestimmung (Schmelztemperaturwert) wie in Beispiel 4 durchgeführt werden. Bei Bedarf kann in einem nächsten Schritt eine genaue Allelbestimmung über eine Sequenzierung erfolgen. Der Vorteil der erfindungsgemäßen Quantifizierung gegenüber unspezifischen SYBR Green/ EtBr-Zumischungen ist unter anderem, dass größere Amplifikate nicht stärker markiert werden ("leuchten"), als kleine Amplifikate, da die Menge des Fluoreszenzfarbstoff über den eingebauten Primer bestimmt wird. Bei unspezifischen Doppelstrangbinden Farbstoffen werden größere Amplifikate stärker markiert (mehr Farbstoff kann angelagert werden), was leicht zu einer Verfälschung bei der Quantifizierung von Templaten führen kann (z.B. unterschiedlich lange SplicingVarianten nach einer reversen Transkription von mRNA). Die erfindungsgemäße Allelbestimmung hat zudem nicht die Einschränkungen des SNUPE-Verfahrens, bei dem die "Fehlstelle" vorher genau bekannt sein muss.

### Erläuterung der Figuren

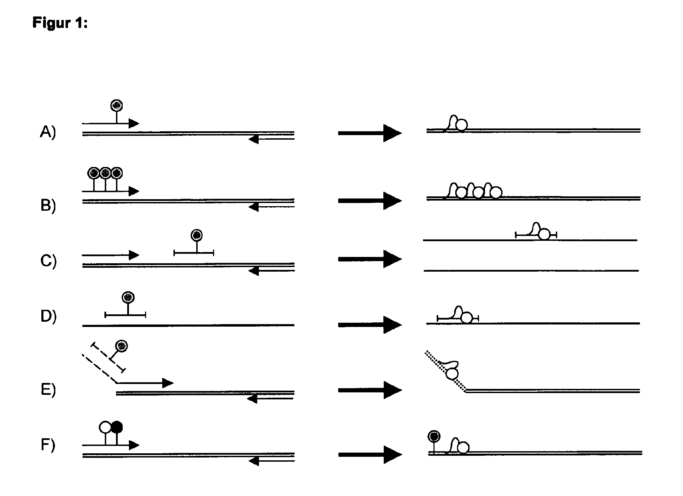

**Figur 1** zeigt Beispiele von möglichen Anwendungen der hier vorgestellten Technologie:
   A) einfach markierter PCR-Primer: Fluoreszenz ist nach Amplifikation/Einbau messbar
   B) mehrfach markierter PCR-Primer: Fluoreszenz ist nach Amplifikation/Einbau messbar
   C) einfach markiert Sonde I: Fluoreszenz ist nach Bindung an Amplifikate messbar
   D) einfach markierte Sonde II: Fluoreszenz ist nach Hybridisierung messbar, in diesem Fall ist keine Amplifikation notwendig.
   E) einfach markierte Sonde III: Fluoreszenz ist nach Hybridisierung an Amplifikat messbar, die Erkennungssequenz entspricht einem Überhang eines Außenprimers.
   F) doppelt markierter PCR-Primer: Anfangsfluoreszenz wird durch Quencherfarbstoff vor Einbau in Amplifikat reduziert, alternativ kann auch eine Fluoreszenzverschiebung mittels eines zweiten Fluorophors (Beispiel TAMRA und Interkalator) hier verwendet werden.
**Figur 2** zeigt Fluoreszenzwerte bei Verwendung einer SYBR^{®} 102-markierten Nukleinsäuresonde als PCR-Außenprimer in Abhängigkeit der Amplifizierungszyklen und bei Verwendung von unterschiedlichen Mischungsverhältnissen der beiden Primer zum Templat (Beispiel 2). Dargestellt sind die ersten 25 Zyklen der Real-Time-PCR. (Die Kurvenbezeichnungen geben das Mischungsverhältnis der beiden Primer und das verwendete Templat an).
**Figur 3** zeigt die Auftrennung der PCR-Ansätze aus Beispiel 2 durch Gelelektrophorese (Agarose-Matrix, Ethidiumbromid-Färbung) der oben beschriebenen PCR-Ansätze. Als Referenz (A/A') wurde ein nicht SYBR^{®} 102-markiertes Primerpaar gleicher Sequenz verwendet.
   - A/A':: entspricht Probe 1/2 jedoch unmarkierte PCR-Primer
   pUC18-Templat
   - B/B':: entspricht Probe 1/2
   pUC18-Templat
   - C/C':: entspricht Probe 3/4
   pUC18-Templat
   - D/D':: entspricht Probe 5/6
   pUC18-Templat
   - E/E':: entspricht Probe 11/12
   pBAD HisA-Templat
**Figur 4** zeigt die Fluoreszenzentstehung in Abhängigkeit der Templatkonzentration, wobei die Nukleinsäuresonde als Primer in einer PCR-Reaktion verwendet wurde. Dargestellt ist die Fluoreszenz-Zunahme der Einzelproben im Laufe der Amplifikation.
**Figur 5** zeigt die Abhängigkeit der Schmelztemperatur von der Anzahl an Fehlern die in dem Doppelstrang vorliegen, in den die erfindungsgemäße Nukleinsäuresonde eingebaut wurde. Aufgetragen ist die relative Fluoreszenz der Proben (in Abhängigkeit von der Temperatur) gegen die Temperatur.
**Figur 6** zeigt zwei Beispiele von modifizierten Basen (1: Uridin-Base, 2: Cytidin-Base) mit Linkereinheiten, die für die Herstellung der erfindungsgemäßen Nukleinsäuresonden verwendet werden können.

### Beispiel 1

### Sequenzen der eingesetzten Oligonukleotide (ODN)

### 1. Interkalator markierter Strang:

M13rev-SYBR^{®}: 5' agc gga t*aa caa ttt cac aca gga 3'
t*: Farbstoffmarkierung mit SYBR^{®} 102 (Invitrogen, Molecular Probes S-21501)

### 2. Gegenstränge (Fehlpaarungen durch * hervorgehoben):

- 0 Fehler:: 5' tcc tgt gtg aaa ttg tta tcc gct 3'
- 1 Fehler:: 5' tcc tgt gtg aat* ttg tta tcc gct 3'
- 2 Fehler:: 5' tcc tgt c*tg aaa ttg a*ta tcc gct 3'
- 3 Fehler:: 5' tcg* tgt gtc* aaa ttg ttt* tcc gct 3'
- 4 Fehler :: 5' tcc c*gt gtg c*aa a*tg tta g*cc gct 3'

- Primer M13fwd:: 5' cgc cag ggt ttt ccc agt cac gac 3'
- Primer M13rev:: 5' agc gga taa caa ttt cac aca gga 3'

### Beispiel 2

### Bestimmung der Spezifität der eingesetzten Nukleinsäuresonden bei Verwendung als PCR-Primer

Durch die SYBR^{®} 102-Farbstoffmarkierung könnte es zu einer Veränderung der Spezifität in Bezug auf die Bindung zwischen Templat und farbstoffmarkiertem Primer kommen, da der eingesetzte PCR-Primer mit einem Doppelstrang-bindenden Farbstoff markiert ist. Um zu überprüfen, ob eine unspezifische Amplifikation auftritt, wird eine Amplifikation mit einem spezifischen und einem unspezifischen Templat (bei konstanter Templat-Konzentration) durchgeführt. Als spezifisches Templat wird hierbei pUC18-Plasmid verwendet, für das die entsprechenden PCR-Primer ausgewählt wurden. Als unspezifisches Templat wird pBAD HisA-Plasmid verwendet, das keine homologen Bereiche für die Amplifikation mit dem eingesetzten Primerpaar aufweist.

Als weiterer Teilversuch wird die Konzentration des Farbstoff-markierten Primers gegenüber dem unmarkierten PCR-Primer variiert, um zu überprüfen, ob durch zufällige Konzentrationsunterschiede bei dem Einsatz SYBR^{®} 102-markierten PCR-Primerpaaren die Spezifität verloren geht und Fluoreszenz, in Abhängigkeit von der Primerkonzentration, und nicht der Spezifität des Templates entsteht. Dies wäre der Fall, wenn durch die Farbstoffmarkierung die Spezifität der auszutestenden Primer stark beeinflusst würde.

### PCR-Protokoll

| | | | | |
|---|---|---|---|---|
| Zyklus 1: | | ( 1X) | | |
| | Step 1: | | 94,0°C | für 03:00 min |
| Zyklus 2: | | ( 50X) | | |
| | Step 1: (Aufschmelzen) | | 96,0°C | für 00:30 min |
| | Step 2: (Anlagerung) | | 48,0°C | für 00:20 min |
| | Step 3: (Verlängerung) | | 72,0°C | für 00:30 min |
| | Datensammlung und real-time-Analyse | | | |

Die Detektion erfolgt bei einer Wellenlänge, bei der die entstehende Fluoreszenz des SYBR^{®}-102 detektiert werden kann (Anregungswellenlänge 494 nm, Messwellenlänge 521 nm). Unterschiedliche Primer-Mischungsverhältnisse bei spezifischen (pUC18) und unspezifischem (pBAD HisA) Templat sehen wie folgt aus:

### Pipettierschema der Proben (als Doppelbestimmungen)

**Tabelle 1:**

| | | | | | | |
|---|---|---|---|---|---|---|
| | pUC18-Plasmid-Templat | | | pBAD HisA-Plasmid-Templat | | |
| Probe | 1/2 | 3/4 | 5/6 | 7/8 | 9/10 | 11/12 |
| M13-rev-SYBR^{®} (100 µM) | 0.5 µl | 1.0 µl | 1.5 µl | 0.5 µl | 1.0 µl | 1.5 µl |
| M13fwd (100 µM) | 0.5 µl | 0.5 µl | 0.5 µl | 0.5 µl | 0.5 µl | 0.5 µl |
| 10x Puffer-Mix* | 5.0 µl | 5.0 µl | 5.0 µl | 5.0 µl | 5.0 µl | 5.0 µl |
| dNTP-Mix (12,5 µM) | 8.0 µl | 8.0 µl | 8.0 µl | 8.0 µl | 8.0 µl | 8.0 µl |
| Templat-Plasmid (ca 15µg/µl) | 1,5 µl | 1,5 µl | 1,5 µl | 1,5 µl | 1,5 µl | 1,5 µl |
| Wasser | 34.0 µl | 33.5 µl | 33.0 µl | 34.0 µl | 33.5 µl | 33.0 µl |
| Taq-Polymerase (5U/µl) | 2.5 U | 2.5 U | 2.5 U | 2.5 U | 2.5 U | 2.5 U |

| | | | | | | |
|---|---|---|---|---|---|---|
| * Taq-spezifischer Puffer (Fa. PeqLab) | | | | | | |

### Ergebnisse

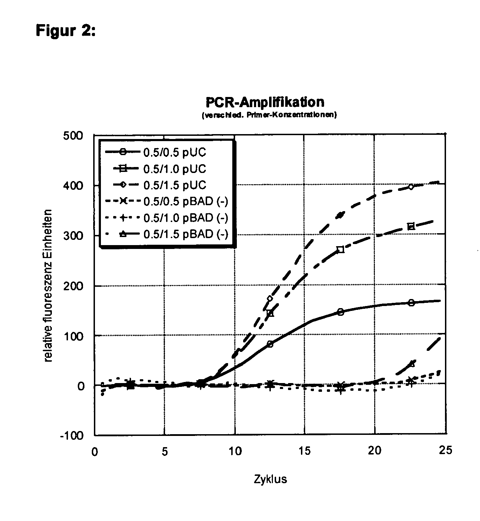

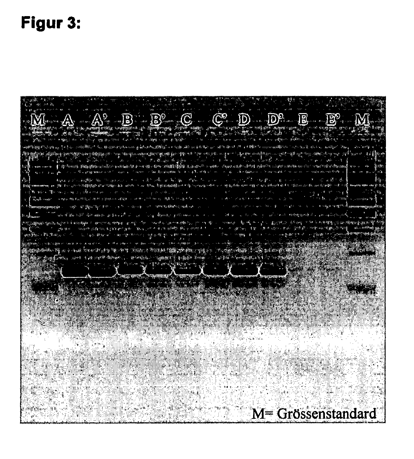

In Abhängigkeit der eingesetzten Konzentration des SYBR^{®} 102-markierten Primers entstehen unterschiedliche hohe Fluoreszenzwerte, da aber immer die gleiche Templatkonzentration (pUC18-Plasmid) eingesetzt wurde, kann bei allen 3 Konzentrationen eine Fluoreszenzentwicklung ab etwa dem siebten Zyklus beobachtet werden, die ab dem 20. Zyklus in eine Plateau-Phase (auf unterschiedlich hohem Niveau) übergeht (siehe Figur 2). Bei dem unspezifischen Templat (pBAD HisA-Plasmid) ist erst nach ca 22-25 Zyklen eine leichte Fluoreszenz messbar. Die Primerkonzentration hat (in den eingesetzten Konzentrationen) nur einen Einfluss auf die maximal erreichbare Fluoreszenz, nicht aber auf den Zeitpunkt der erstmaligen Fluoreszenzentwicklung. Das heißt, ein entsprechend SYBR^{®}-markierter Primer kann zur spezifischen Detektion der entsprechenden DNA-Sequenz verwendet werden, wobei die Konzentration der eingesetzten Primer nur Einfluss auf die Signalhöhe, nicht aber auf das Signal selber hat. Die Spezifität des eingesetzten Primerpaares wird durch die SYBR^{®} 102-Markierung nicht herabgesetzt. Das unspezifischen Templat wird erst nach einer viel höheren Anzahl von PCR-Zyklen amplifiziert.
Dieses Ergebnis zeigt sich auch, wenn entsprechende Amplifikate in einer Gelektrophorese aufgetrennt werden (siehe Figur 3).

### Beispiel 3

### Bestimmung der Empfindlichkeit der eingesetzten Nukleinsäuresonden bei Verwendung als PCR-Primer

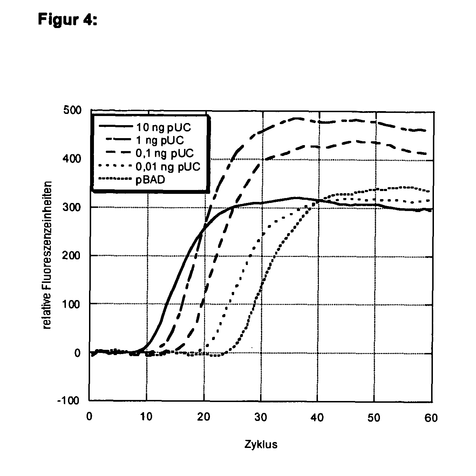

Da die untersuchten Primer aus Beispiel 2 spezifisch eine bestimmte Sequenz amplifizieren, ist es möglich, über den Zeitpunkt der auftretenden Fluoreszenz eine Mengenbestimmung des eingesetzten Templates durchzuführen. Dazu wurden von dem spezifischen Templat (pUC18) unterschiedliche Mengen zur Amplifikation eingesetzt, und als Vergleich die höchste Konzentration des unspezifischen Templates (pBAD HisA) (siehe Figur 4).

### Pipettierschema der Proben (Doppelbestimmungen)

| | | | | | |
|---|---|---|---|---|---|
| | pUC18-Plasmid-Templat | | | | pBAD HisA |
| Probe | 1/2 | 3/4 | 5/6 | 7/8 | 9/10 |
| M13revSYBR^{®} (100 µM) | 1.0 µl | 1.0 µl | 1.0 µl | 1.0 µl | 1.0 µl |
| M13fwd (100 µM) | 0.5 µl | 0.5 µl | 0.5 µl | 0.5 µl | 0.5 µl |
| 10x Puffer-Mix* | 5.0 µl | 5.0 µl | 5.0 µl | 5.0 µl | 5.0 µl |
| dNTP-Mix (12,5 µM) | 8.0 µl | 8.0 µl | 8.0 µl | 8.0 µl | 8.0 µl |
| Templat-Plasmid (variabel) | 1,5 µl | 1,5 µl | 1,5 µl | 1,5 µl | 1,5 µl |
| Wasser | 33.5 µl | 33.5 µl | 33.5 µl | 33.5 µl | 33.5 µl |
| Taq-Polymerase (5U/ µl) | 2.5 U | 2.5 U | 2.5 U | 2.5 U | 2.5 U |

| | | | | | |
|---|---|---|---|---|---|
| * Taq-spezifischer Puffer (Fa. PeqLab) | | | | | |

Bemerkung: 1/2: 10.00 ng/µl pUC18-Plasmid, 3/4: 1.00 ng/ µl pUC18-Plasmid, 5/6: 0.10 ng/ µl pUC18-Plasmid, 7/8: 0.01 ng/µl pUC18-Plasmid, 9/10: 10 ng/µl pBAD HisA-Plasmid (unspezifisches Templat)

### Ergebnisse

Deutlich zu erkennen ist, dass die Fluoreszenzentstehung an die Templatmenge gebunden ist (siehe Figur 4). Innerhalb einer bestimmten Zyklenzahl entsteht ein fluoreszierender DNA-Doppelstrang, jedoch nur wenn die eingesetzten Primer spezifisch für dieses Templat konzipiert wurden. Je höher die Templatkonzentration ist, (10 ng gegenüber 0,01 ng) desto früher entwickelt sich eine messbare Fluoreszenz (ca. 8. Zyklus (10 ng Templat) → ca. 20. Zyklus (0,01 ng Templat)). Wird als Templat eine unspezifische DNA (z.B. pBAD HisA-Plasmid mit einer nicht komplementären Sequenz) eingesetzt, so entsteht erst sehr viel später eine messbare Fluoreszenz (ab Zyklus 25 gegenüber Zyklus 8 bei gleicher Templat-Konzentration), bzw. wenn man die Templatmengen extrapoliert, müsste man die 10,000 fache Menge unspezifisches Templat gegenüber spezifischen Templat einsetzen, um bei der gleichen Zyklenzahl eine Fluoreszenz zu detektieren. Das heißt durch die interne SYBR^{®} 102-Markierung geht die Spezifität der Sonde nicht verloren.
Eine SYBR^{®} 102-Markierung kann also eingesetzt werden, um die Häufigkeit (Quantität) einer Sequenz (z.B. in einer Mischung, oder in einer Probe) zu detektieren. Anhand von einer Eichgeraden könnte auf diese Art und Weise eine Konzentrationsbestimmung vorgenommen werden. Aber die Markierung kann damit auch zum Einsatz kommen, wenn es um die Typisierung von DNA geht (Dabei kann spezifische DNA aus einem Pool von unspezifischen DNA-Sequenzen amplifiziert werden).
Nur wenn die zu untersuchende Test-DNA die Zielsequenz enthält, entsteht eine messbare Fluoreszenz innerhalb einer bestimmten Zyklenzahl. Es kann also mit der SYBR® 102-Markierung sowohl der Nachweis, als auch eine Quantifizierung vorgenommen werden.

### Beispiel 4

### Bestimmung von Schmelzpunktskurven von SYBR^{®} 102-markierten Oligonukleotiden

Der hier beschriebene Versuch soll veranschaulichen, dass es durch die von uns entwickelte Sondentechnik möglich ist, Punktmutationen innerhalb der Sonden/Bindungssequenz nachzuweisen.

Der Schmelzpunkt bzw. die thermische Stabilität einer DNA-Doppelhelix ist unter anderem abhängig von der Anzahl der Fehlpaarungen, die innerhalb eines Doppelstranges vorliegen. Da der eingesetzte Farbstoff nur fluoresziert, wenn er an Nukleinsäuredoppelstränge gebunden hat, ist es möglich anhand der gemessenen Fluoreszenz Aussagen über das strukturelle Vorliegen der Sonde zu treffen (Doppelstrang, partieller Doppelstrang, Einzelstrang).

Für die Untersuchung wurden Strang (Primer M13revSYBR^{®}, SYBR^{®}102-markiert) und Gegenstrang (Gegenstränge 0 bis 4 Fehler, unspezifisch pBAD HisA-Plasmid, alle Gegenstränge jeweils unmarkiert) äquimolar gemischt und bei gleichzeitiger Detektion der Fluoreszenz erwärmt (Anregungswellenlänge 494 nm, Messwellenlänge 521 nm).

### Pipettierschema der Proben

| | | | | | | | |
|---|---|---|---|---|---|---|---|
| Probe | 0 | 1 | 2 | 3 | 4 | pBAD | ohne |
| | Fehler | Fehler | Fehler | Fehler | Fehler | HisA | Gegenstrang |
| SYBR^{®}-Primer | 0,5 µl | 0,5 µl | 0,5 µl | 0,5 µl | 0,5 µl | 0,5 µl | 0,5 µl |
| (100 µM) | | | | | | | |
| Gegenstrang | 0,5 µl | 0,5 µl | 0,5 µl | 0,5 µl | 0,5 µl | pBAD | -------- |
| (100 µM) | | | | | | HisA | |
| 10x Puffer* | 5 µl | 5 µl | 5 µl | 5 µl | 5 µl | 5 µl | 5 µl |
| Wasser | 44 µl | 44 µl | 44 µl | 44 µl | 44 µl | 44 µl | 44.5 µl |

| | | | | | | | |
|---|---|---|---|---|---|---|---|
| * Taq-spezifischer Puffer (Fa. PeqLab) | | | | | | | |

### Ergebnisse

In Figur 5 kann man deutlich erkennen, dass die Anzahl der Fehler einen Einfluss auf die Schmelztemperatur der untersuchten Doppelstränge hat, wobei die SYBR^{®} 102-Markierung zur Detektion des Schmelzverhaltens herangezogen werden kann. Je geringer die Anzahl der Fehlpaarungen ist, desto stärker müssen die Doppelstränge aufgeheizt werden, um eine Abnahme der Fluoreszenz zu erreichen. Man kann auf diese Art und Weise über die Temperatur (oder einen zu definierenden Schwellenwert, siehe waagerechter Strich in der Graphik) Rückschlüsse auf die Passgenauigkeit der beiden DNA-Stränge ziehen. Je besser die beiden Stränge zusammenpassen, desto höher ist die erforderliche Temperatur.
Damit können SYBR® 102-markiert Sonden eingesetzt werden, um Punktmutationen zu detektieren bzw. um DNA unbekannter Herkunft zu typisieren.

### Annex zu den Anmeldungsunterlagen - nachgereichtes Sequenzprotokoll

## Patentansprüche

1. Nukleinsäuresonde, ausgewählt aus der Gruppe bestehend aus DNA, RNA, LNA, CNA, HNA, ANA, FANA und Chimären aus diesen, worin die Nukleinsäuresonde weitere Modifikationen am Zucker, an der Base oder am Phosphatrückgrat aufweisen kann, und worin eine oder mehrere nicht endständige Basenpositionen der Nukleinsäuresonde durch eine homologe Base ersetzt sind, an die ein doppelstrang-bindender Fluoreszenzfarbstoff kovalent über eine Linkereinheit gebunden ist

2. Nukleinsäuresonde nach Anspruch 1, worin zusätzlich mindestens ein Fluorophor kovalent an die Nukleinsäuresonde gebunden ist.

3. Nukleinsäuresonde nach Anspruch 1 oder 2, worin 2-4 Basenpositionen der Nukleinsäuresonde durch eine homologe Base ersetzt sind, an die ein doppelstrang-bindender Fluoreszenzfarbstoff über eine Linkereinheit kovalent gebunden ist.

4. Nukleinsäuresonde nach einem der vorangegangenen Ansprüche, worin die Nukleinsäuresonde aus Chimären von DNA, RNA, LNA, CNA, HNA, ANA und FANA besteht.

5. Nukleinsäuresonde nach einem der vorangegangenen Ansprüche, worin die Nukleinsäuresonde kein peptidisches Rückgrat besitzt.

6. Nukleinsäuresonde nach einem der der vorangegangenen Ansprüche, worin das 3'- oder 5'-Ende der Nukleinsäuresonde eine Affinitätsmarkierung aufweist.

7. Nukleinsäuresonde nach Anspruch 6, worin die Affinitätsmarkierung ein kovalent gebundenes Biomolekül darstellt, ausgewählt aus der Gruppe bestehend aus Antigenen, Antikörper, Proteinen, Lecitinen, Enzymen und Coenzymen.

8. Nukleinsäuresonde nach einem der vorangegangenen Ansprüche, worin die Nukleinsäuresonde mit Fluoreszenzfarbstoffen markiert ist, ausgewählt aus der Gruppe bestehend aus SYBR® 101, SYBR® 102, SYBR® 103, Pyren-Derivaten, Ethidiumbromid-Derivaten oder Acridin-Derivaten.

9. Nukleinsäuresonde nach einem der vorangegangenen Ansprüche, worin die Fluoreszenzfarbstoffe ausgewählt sind aus der Gruppe bestehend aus SYBR^{®} 101, SYBR^{®} 102 und SYBR^{®} 103.

10. Verfahren zur Hybridisierung einer Nukleinsäuresonde mit einer Nukleinsäureprobe, umfassend die Schritte von:
a) dem Bereitstellen einer Nukleinsäuresonde nach einem der Ansprüche 1 bis 9,
b) dem in Kontaktbringen der Nukleinsäureprobe mit der Nukleinsäuresonde zur Hybridisierung der Nukleinsäureprobe mit der Nukleinsäuresonde,
c) dem Messen der Änderung des Fluoreszenzverhaltens.

11. Verfahren nach Anspruch 10, worin die Nukleinsäureprobe ausgewählt ist aus der Gruppe, bestehend aus DNA, RNA, LNA und PNA.

12. Verfahren nach einem der vorangegangenen Ansprüche, worin verschiedene Farbstoffe bei unterschiedlichen Fluoreszenzwellenlängen detektiert werden können.

13. Verfahren nach einem der Ansprüche 10 bis 12, worin die Hybridisierung der Nukleinsäuresonde mit der Nukleinsäureprobe in einem PCR (Polymerase-Ketten-Reaktion)-Verfahren zur Amplifikation der Nukleinsäureprobe erfolgt.

14. Verfahren nach einem der Ansprüche 10 bis 13, worin die Nukleinsäuresonde als nicht verlängerbarer Innenprimer verwendet wird.

15. Verfahren nach einem der Ansprüche 10 bis 13, worin die Nukleinsäuresonde als Außenprimer verwendet wird.

16. Verfahren nach Anspruch 15, worin mindestens 2 Außenprimer-Paare verwendet werden, wobei mindestens ein Primer des Außenprimer-Paares eine farbstoffmarkierte Nukleinsäuresonden ist, und wobei die Außenprimer-Paare jeweils unterschiedliche Farbstoffe aufweisen.

17. Verfahren nach Anspruch 10 bis 14, worin mindestens zwei Nukleinsäuresonden nach einem der Ansprüche 1 bis 9 in dem Verfahren verwendet werden.

18. Verfahren nach einem der Ansprüche 10 bis 12, worin die Hybridisierung der Nukleinsäuresonde mit der Nukleinsäureprobe zur direkten Detektion von Nukleinsäureproben verwendet wird.

19. Verfahren nach einem der Ansprüche 10 bis 18, worin ein Gemisch aus verschiedenen Nukleinsäuresonden verwendet wird, die sich in ihrem Fluoreszenzverhalten unterscheiden, wobei dieses Gemisch auch nicht erfindungemäße Nukleinsäuresonden umfassen kann, und worin ein Gemisch aus Nukleinsäureproben verwendet wird, sodass je eine spezifische Nukleinsäuresonde mit je einer spezifischen Nukleinsäureprobe hybridisieren kann und wobei weitere unspezifische Nukleinsäureproben in dem Gemisch enthalten sein können.

20. Verfahren nach einem der Ansprüche 10 bis 19, worin eine oder mehrere Nukleinsäuresonden auf einem festen Träger (Chip) immobilisiert sind.
